(19) Europäisches Patentamt
European Patent Office
Office européen des brevets

(11) EP 4 115 953 A1

(12) **EUROPEAN PATENT APPLICATION**
published in accordance with Art. 153(4) EPC

(43) Date of publication:
**11.01.2023 Bulletin 2023/02**

(21) Application number: **21765051.4**

(22) Date of filing: **04.03.2021**

(51) International Patent Classification (IPC):
***A61Q 19/00*** *(2006.01)* ***A61K 8/06*** *(2006.01)*
***A61K 8/34*** *(2006.01)* ***A61K 8/894*** *(2006.01)*

(52) Cooperative Patent Classification (CPC):
**A61K 8/06; A61K 8/34; A61K 8/894; A61Q 19/00**

(86) International application number:
**PCT/JP2021/008412**

(87) International publication number:
**WO 2021/177400 (10.09.2021 Gazette 2021/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.03.2020 JP 2020036519**

(71) Applicant: **Shiseido Company, Ltd.**
**Chuo-ku**
**Tokyo 104-0061 (JP)**

(72) Inventors:
- **UENO Kento**
  **Tokyo 104-0061 (JP)**
- **INOUE Haruhiko**
  **Tokyo 104-0061 (JP)**
- **MUNAKATA Hidehito**
  **Tokyo 104-0061 (JP)**
- **WATANABE Kei**
  **Tokyo 104-0061 (JP)**

(74) Representative: **Ter Meer Steinmeister & Partner Patentanwälte mbB**
**Nymphenburger Straße 4**
**80335 München (DE)**

(54) **OIL-IN-WATER EMULSION COMPOSITION**

(57) An object of the present invention is to provide, regarding a composition containing a nanodisc composed of a silicone-based surfactant, a nanodisc-containing composition having an improved stability of an emulsion composition.

For achieving the object, provided is an oil-in-water emulsion composition containing (A) an aqueous phase, (B) an oil phase, and (C) polyoxyalkylene-modified silicone. When the oil-in-water emulsion composition contains 1 to 35% by mass in total of ethyl alcohol and dipropylene glycol in (A) the aqueous phase, 1 to 50% by mass of (B) the oil phase, and 0.2 to 5% by mass of (C) based on the whole composition, a nanodisc is formed and the emulsion state is stable.

[FIG. 8]

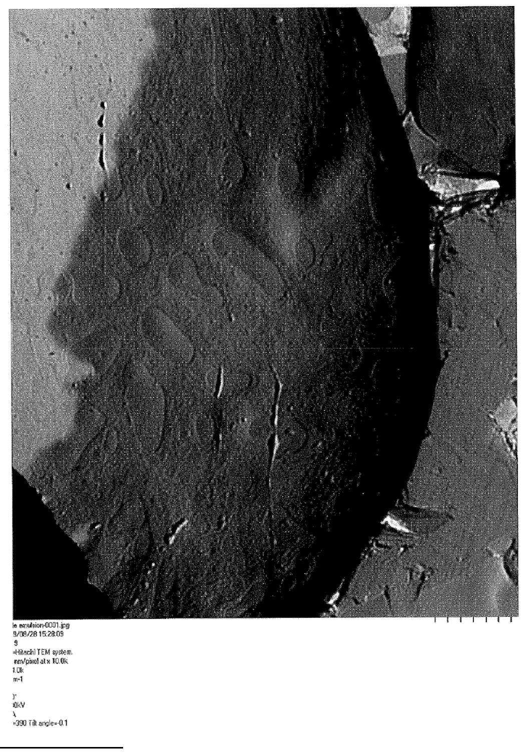

EP 4 115 953 A1

## Description

RELATED APPLICATION

The present invention claims a priority based on the Japanese Patent Application No. 2020-036519 (filed on March 4, 2020), and the entire contents described in the same application are deemed to be incorporated herein by reference.

FIELD OF THE INVENTION

The present invention relates to a nanodisc emulsion composition and a method for producing the same, and in particular, to the enhancement in the emulsion stability and usability of the emulsification of a nanodisc emulsion composition containing a silicone-based surfactant.

BACKGROUND OF THE INVENTION

Among amphipathic compounds having both hydrophilic and hydrophobic properties, some compounds, like phospholipids, form a spherical endoplasmic reticulum composed of a bilayer membrane (lamellar phase) in an aqueous phase. Such a bilayer membrane endoplasmic reticulum is called liposome or vesicle, and these can stably retain an aqueous component inside the endoplasmic reticulum, or can stably retain an oily component in the endoplasmic reticulum membrane. For this reason, for example, when these are allowed to retain a drug and be administered to the body, there are benefits such as the metabolism being suppressed and thus the drug efficacy being maintained for an extended period of time. Consequently, these compounds have been used as microcapsules in the fields of medicines, cosmetics, foods, and the like. On the other hand, a nanodisc which does not includes an internal phase is a plate-like dispersion of a lamellar liquid crystal phase. Such a nanodisc can stably retain an oily component in the endoplasmic reticulum membrane but does not include an internal phase.

Patent Literatures 1 and 2 disclose the use of a specific polyoxyethylene hydrogenated castor oil derivative as an amphipathic substance to form a vesicle, which is contained as an emulsifier, whereby a cosmetic which is not sticky and has a good feel when used was obtained.

Additionally, a silicone-based surfactant is documented as such an amphipathic compound capable of forming a vesicle (e.g., see Patent Literatures 3 to 7). Examples of the features of the vesicle formed by a silicone-based surfactant include possible easier preparation of a vesicle when compared with the case where other surfactants having a vesicle-forming ability are used.

Patent Literature 7 discloses a technology for dispersing a water-insoluble liquid phase in an external phase by a vesicle including an internal phase. However, the emulsification by a vesicle is unstable and limited in practical use. For this reason, the amount of a vesicle added tends to be large, sometimes posing a problem of stickiness and the like caused by a vesicle-forming surfactant as generally has been known. Further, no report has been documented on the formation of a nanodisc using a silicone-based surfactant.

[Patent Literature 1]
International Publication No. WO2010-064678
[Patent Literature 2]
Japanese Patent Laid-Open No. 2011-195509
[Patent Literature 3]
Japanese Patent Laid-Open No. 07-323222
[Patent Literature 4]
Japanese Patent Laid-Open No. 08-239475
[Patent Literature 5]
Japanese Patent Laid-Open No. 09-175930
[Patent Literature 6]
Japanese Patent No. 5121179
[Patent Literature 7]
Japanese Patent No. 3137592

[Non Patent Literature 1]

H. SAGITANI, Y. HIRAI, K. NABETA and M. NAGAI, Effect of Types of Polyols on Surfactant Phase Emulsification, J. Jpn Oil Chem. Soc., Vol. 35, 102-107 (1986)
[Non Patent Literature 2]
Kei Watanabe, Miharu Nishida, Kanako Nishimura, Yoriko Mune, Yuji Matsushita, Ayano Nakamura, Koji Tsuchiya, Hideki Sakai, Heinz Hoffmann, High Skin Hydration and Comfortable Texture of a Moisturizing Lotion Fulfilled by Controlling the Phase Sequence of a Vesicle/Micelle Complex, J. Soc. Cosmet. Chem. Jpn., 52, (4) 260-268 (2018)

DISCLOSUER OF THE INVENTION

PROBLEM TO BE SOLVED BY THE INVENTION

Conventional vesicle emulsions have not been necessarily sufficient in the stability with the passage of time or temperature. The present invention was carried out in light of the problems of conventional technology, and an object thereof is to improve the stability of an emulsion composition by a nanodisc, not by the vesicle which includes an internal phase.

MEANS TO SOLVE THE PROBLEM

The present inventors carried out extensive studies for solving the problems of conventional technology, and have consequently found that the emulsion stability can be retained in the oil-in-water emulsion composition comprising an aqueous phase, an oil phase, and a specific silicone-based surfactant, when a silicone nanodisc, which does not include an internal phase, formed by optimizing formation conditions of a vesicle to be a nanodisc precursor is adsorbed to the oil-water interface, whereby the present invention has been accomplished.
The oil-in-water emulsion composition of the present invention is an oil-in-water emulsion composition comprising (A) an aqueous phase, (B) an oil phase, and (C) polyoxyalkylene-modified silicone, wherein the oil-in-water emulsion composition comprises:

1 to 35% by mass in total of a monohydric alcohol and a dihydric glycol in (A) the aqueous phase, with the monohydric alcohol alone in a range of 1 to 15% by mass, and the dihydric glycol alone in a range of 1 to 20% by mass;
1 to 50% by mass of (B) the oil phase; and
0.2 to 5% by mass of (C) based on the whole composition.

The monohydric alcohol in (A) the aqueous phase is preferably ethyl alcohol, and the dihydric glycol is preferably dipropylene glycol.
Further, the proportion of a silicone oil in (B) the oil phase is 50% by mass or less, and the component (C) is PEG-12 dimethicone. Additionally, PEG-12 dimethicone in a concentration of 5 to 20% by mass does not dissolve but precipitates in water, and has an HLB of less than 10 as calculated by Griffin's formula.
When the emulsion composition is centrifuged at 40000 rpm for 60 minutes, particles having an average particle size of 30 nm to 150 nm are present in a clear layer separated to a lower layer; and when the emulsion composition is centrifuged at 3000 rpm for 16 hours, a clear separated layer of an oil at a proportion of 2% in the total volume is not observed in an upper layer or the lower layer.
In the oil-in-water emulsion composition, a lamellar nanodisc is adsorbed to an oil-water interface. The nanodisc has a major axis ranging from 20 nm to 1000 nm.
The oil-in-water emulsion composition may comprise, as a component (D), one or two or more ionic surfactants selected from a sulfosuccinic acid diester salt, an alkyl aryl sulfonic acid salt, an alkyl ether sulfonic acid salt, a sulfosuccinic acid ester salt, an acyl methyltaurine salt, and an acyl taurine, and particularly preferable is an N-stearoyl-N-methyltaurine salt.
The content of (D) the ionic surfactant may be 0.01 to 1.0% by mass based on the whole oil-in-water emulsion composition.
Additionally, the oil-in-water emulsion composition may comprise, as a component (E), a polymer thickening agent in a concentration of 0.05 to 1% by mass. The component (E) is preferably a carboxyvinyl polymer or a derivative thereof, or an acrylic thickening agent. When the polymer thickening agent as the component (E) is an acrylic thickening agent, the acrylic thickening agent is one or two or more acrylic thickening agents selected from a (dimethylacrylamide/sodium acryloyldimethyltaurate) crosspolymer, an ammonium acryloyldimethyltaurate/VP copolymer, an (ammonium acryloyldimethyltaurate/beheneth-25 methacrylate) crosspolymer, and a (sodium acrylate/sodium acryloyldimethyltaurate) copolymer.
Further, the oil-in-water emulsion composition may comprise an elastomer (F).

## EFFECT OF THE INVENTION

The nanodisc-containing composition according to the present invention containing (A) the aqueous phase, (B) the oil phase, and (C) polyoxyalkylene-modified silicone in a specific amount has enhanced emulsion stability and good usability.

## BRIEF DESCRIPTION OF THE DRAWINGS

[FIG. 1] FIG. 1 is a drawing showing a vesicle forming region by HLB of PEG-12 dimethicone when a PEG-12 dimethicone concentration is 1% by mass and the ethyl alcohol concentration.
[FIG. 2] FIG. 2 is a drawing showing the particle size peak of the particles comprised in the aqueous phase before the emulsification in a undissolved state (an alcohol amount is 0% by mass).
[FIG. 3] FIG. 3 is a drawing showing the particle size peak of the particles comprised in the aqueous phase at the lower layer after the emulsion emulsified in a undissolved state (an alcohol amount is 0% by mass, an oil amount is 10% by mass) was centrifuged at 40000 rpm for 60 minutes.
[FIG. 4] FIG. 4 is a drawing showing the particle size peak of the particles comprised in the aqueous phase before the emulsification in a micelle state (an alcohol amount is 20% by mass).
[FIG. 5] FIG. 5 is a drawing showing the particle size peak of the particles comprised in the aqueous phase at the lower layer after the emulsion emulsified in a micelle status (an alcohol amount is 20% by mass, an oil amount is 10% by mass) was centrifuged at 40000 rpm for 60 minutes.
[FIG. 6] FIG. 6 is a drawing showing the particle size peak of the particles comprised in the aqueous phase before the emulsification in a vesicle state (an alcohol amount is 10% by mass).
[FIG. 7] FIG. 7 is a drawing showing the particle size peak of the particles comprised in the aqueous phase at the lower layer after the emulsion emulsified in a state changed from a vesicle to a nanodisc (an alcohol amount is 10% by mass, an oil amount is 10% by mass) was centrifuged at 40000 rpm for 60 minutes.
[FIG. 8] FIG. 8 is an electron micrograph of the nanodisc emulsion at the interface between an oil particle and water.
[FIG. 9] FIG. 9 is an electron micrograph of enlarged nanodiscs of the nanodisc emulsion adsorbed to the interface.
[FIG. 10] FIG. 10 is a schematic drawing showing that a vesicle transitions to a nanodisc at the interface and adsorbs thereto.

## BEST MODE FOR CARRYING OUT THE INVENTION

The nanodisc-containing composition according to the present invention contains (A) an aqueous phase, (B) an oil phase, and (C) polyoxyalkylene-modified silicone. Hereinafter, each component will be described in detail.

The nanodisc of the present invention is in the form of a vesicle, which is the precursor of the nanodisc, in a composition which does not comprise an oil. This vesicle is not a spontaneous vesicle. The spontaneous vesicle refers to a solution in an equilibrium state, that is, a solution preserved at a constant temperature and a constant pressure for an extremely extended period of time is in a state in which vesicles are dispersed. In the vesicle of the present invention, the equilibrium state of a solution is a two-phase coexistence solution of plate-like lamellar liquid crystals and water. When an intense stirring force is applied to this state to disperse, vesicles are formed. When an oil is added to the state of vesicle to carry out emulsification, vesicles undergo the structural transition to nanodiscs. Further, the addition of an ionic surfactant as a dispersing agent enables this condition to be maintained over an extended period of time. Thus, the present invention is accomplished.

The oil-in-water emulsion composition by the adsorption of nanodiscs of the present invention comprises a monohydric alcohol or a dihydric glycol. Examples of the monohydric alcohol include ethyl alcohol, normal propyl alcohol, and isopropyl alcohol. Examples of the dihydric glycol include 1,3-butylene glycol, and dipropylene glycol. These allow, by the solvent effects, a surfactant containing polyether-modified silicone to transform to be hydrophilic (Non Patent Literature 1). As a result, the transition from the vesicle, which is a spherical endoplasmic reticulum, to a nanodisc facilitates. The vesicle, which is a spherical endoplasmic reticulum, has the entire surface covered with a hydrophilic group, but a nanodisc has a lipophilic group at the edge parts thus making it difficult to generate in water. The monohydric alcohol and the dihydric glycol hydrophilize a surfactant by the solvent effects thus making it easier to transition to a nanodisc. On the other hand, when PEG-12 dimethicone is dissolved in alcohol, trihydric glycerin, tetrahydric sorbitol and the like lipophilize a surfactant and inhibit the transition to a nanodisc, hence not desirable, and the content is desirably (the gross amount of the monohydric alcohol and the dihydric glycol)>(the gross amount of the trihydric glycerin and the tetrahydric sorbitol).

(A) Aqueous phase

In the aqueous phase, the total content of the monohydric alcohol and the dihydric glycol in the aqueous phase may be 1 to 45% by mass, and preferably 1 to 35% by mass, and it is preferable that the monohydric alcohol alone is in a range of 1 to 15% by mass, and the dihydric glycol alone is in a range of 1 to 20% by mass. The monohydric alcohol is preferably ethyl alcohol. The dihydric glycol is preferably dipropylene glycol. It is more preferable to contain ethyl alcohol and dipropylene glycol in the upper limit concentrations that satisfy the following [Formula 1].

[Formula 1]

Ethyl alcohol concentration (% by mass) in aqueous phase/15+ dipropylene glycol concentration in aqueous phase (% by mass)/20$\leq$1

When a content of ethyl alcohol alone, a content of dipropylene glycol alone, and the total content of ethyl alcohol and dipropylene glycol are less than 1% by mass, a vesicle may not be generated or the structure is disturbed and the emulsification may fail. When a content of ethyl alcohol alone is more than 15% by mass, when a content of dipropylene glycol alone is more than 20% by mass, further when the content ratio of ethyl alcohol to dipropylene glycol is outside the range of the above [Formula 1], or even when the content ratio is within the range of the above [Formula 1] but the total amount is more than 35% by mass, the vesicle membrane may become too flexible, or a vesicle transitions to a micelle, thereby failing to obtain the stabilization effect.

(B) Oil phase

The oil that can be contained in the oil phase is not particularly limited, and examples include silicone oils (e.g., dimethyl polysiloxane, diphenyl polysiloxane, octamethylcyclotetrasiloxane, decamethylcyclopentasiloxane, decamethylcyclohexasiloxane, amino-modified polysiloxane, polyether-modified polysiloxane, alkyl-modified polysiloxane, and fluoride-modified polysiloxane); hydrocarbon oils (e.g., liquid paraffin, ozokerite, squalane, vaseline, and microcrystalline wax); ester oils (e.g., isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, acetylated lanolin, isocetyl stearate, isocetyl isostearate, cholesteryl 12-hydroxystearate, ethylene glycol di-2-ethylhexanoate, dipentaerythritol fatty acid ester, N-alkyl glycol monoisostearate, neopentyl glycol dicaprate, diisostearyl malate, glycerin di-2-heptylundecanoate, trimethylolpropane tri-2-ethylhexanoate, trimethylolpropane triisostearate, glycerin trioctanoate, glycerin triisopalmitate, trimethylolpropane triisostearate, cetyl-2-ethylhexanoate, 2-ethylhexyl palmitate, glycerin trimyristate, glyceride tri-2-heptylundecanoate, castor oil fatty acid methyl ester, oleyl oleate, acetoglyceride, 2-heptylundecyl palmitate, di-2-heptylundecil adipate, diisobutyl adipate, 2-octyldodecyl N-lauroyl-L-glutamate ester, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, and triethyl citrate).

The oil phase is preferably 1 to 50% by mass based on the whole emulsion.

Further, the content of the silicone oil in the oil phase is preferably 50% by mass or less. When more than 50% by mass of the silicone oil is comprised, emulsion particles can coalesce at a high temperature.

(C) Polyoxyalkylene-modified silicone is a water-soluble silicone-based surfactant in which a moiety of the methyl group in dimethicone is substituted with polyethylene glycol. Such surfactant is excellent in the emulsifying action, dispersing action, and permeating action, and commonly used in the field of cosmetics and represented by the following formula (1).

[Formula 1]

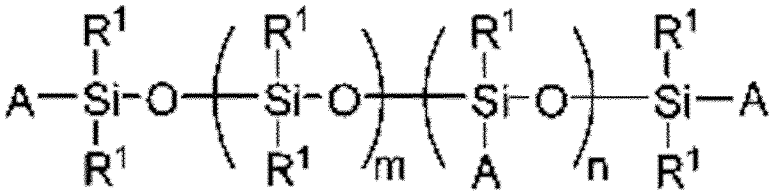

(1)

wherein $R^1$ is hydrogen or an alkyl group having 1 to 6 carbon atoms. At least one of A is a polyoxyalkylene group represented by a formula: $-(CH_2)_a-(C_2H_4O)_b-(C_3H_6O)_c-R^2$ (wherein $R^2$ is hydrogen or an alkyl group having 1 to 6 carbon

atoms, a is an integer of 1 to 6, b is an integer of 0 to 50, c is an integer of 0 to 50, b+c is at least 5 or more), and other A are hydrogen or an alkyl group having 1 to 6 carbon atoms. m is an integer of 1 to 200, and n in an integer of 0 to 50.

Of (C) polyoxyalkylene-modified silicone, particularly preferable is PEG-12 dimethicone wherein c is 0, and b is 12, in [Formula 1].

Examples of the commercial product of PEG-12 dimethicone include DOWSIL ES-5373 (manufactured by Dow Toray Co., Ltd.), SH3772M, SH3773M, SH3775M (all manufactured by Dow Toray Co., Ltd.), and IM-22 (manufactured by Wacker Chemical AG).

The content of the component (C) needs to be an amount capable of forming vesicles, which are the precursor of nanodiscs, and is 0.2 to 5.0% by mass, and more preferably 0.5 to 2.5% by mass, based on the whole composition. When the content is less than 0.2% by mass, the effect by nanodiscs may not be obtained, whereas an amount of more than 5.0% by mass may cause poor stability of nanodiscs.

The oil-in-water emulsion composition according to the present invention contains nanodiscs composed of the surfactant of the component (C). The vesicle, which is the precursor of a nanodisc, can be formed by a known method and, for example, (A) the aqueous phase and the component (C) are mixed and stirred to form vesicles composed of the component (C) in the aqueous phase. The average particle size of vesicles is about 30 nm to 150 nm.

The oil-in-water emulsion composition according to the present invention may further contain further (D) ionic surfactant. When an ionic surfactant is added, the stability of the nanodisc-containing composition containing (C) polyoxyalkylene-modified silicone is improved.

The ionic surfactant used in the present invention can be a surfactant, which is other than (C) the silicone-based surfactant and exhibits the ionicity, and can be used without being limited.

The content of (D) the ionic surfactant is preferably 0.01 to 1.0% by mass, and further preferably 0.01 to 0.1% by mass, based on the whole composition. When the content of the surfactant is small, the stabilization effect on nanodiscs may not be sufficiently obtained, whereas a content that is too large may rather be detrimental such as solubilizing a vesicle, which is the precursor of a nanodisc, or inhibiting the nanodisc formation.

Further, the content ratio of (C) polyoxyalkylene-modified silicone to the ionic surfactant is preferably 1:0.01 to 1:0.1.

An anionic surfactant can be contained as (D) the ionic surfactant that can be contained in the present invention, and when a Krafft point of an anionic surfactant is low (e.g., a temperature lower than room temperature), the silicone-based surfactant and an anionic surfactant are easily mixed and interact, thereby inhibiting the transition from a vesicle to a nanodisc. This is because the anionic surfactant has the nature of forming a spherical aggregate called a micelle and thus has the effect to maintain the spherical structure when coexists with a vesicle, whereby the transition to a nanodisc is inhibited.

For (D) the ionic surfactant that can be contained in the present invention, a sulfonate anionic surfactant is preferable among the anionic surfactants. Examples of the sulfonate anionic surfactant include a sulfosuccinic acid diester salt, an alkyl aryl sulfonic acid salt, an alkyl ether sulfonic acid salt, a sulfosuccinic acid ester salt, an acyl methyltaurine salt, and an acyl taurine salt.

In the present invention, an N-acyl methyltaurine salt is particularly preferably contained as the ionic surfactant. Further, an N-stearoyl-N-methyltaurine salt is preferable among the N-acyl methyltaurine salts represented by the following formula (2).

[Formula 2]

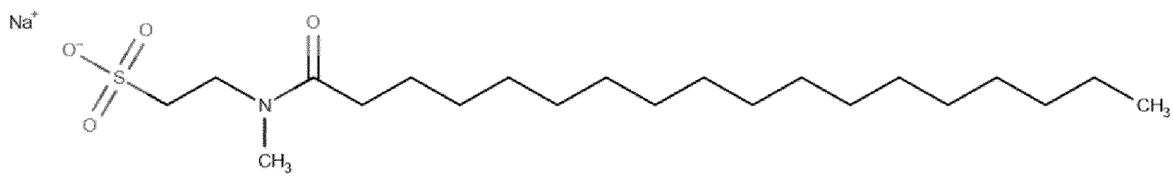

(2)

In the present invention, (E) a polymer thickening agent may further be contained. (E) The polymer thickening agent is preferably a carboxyvinyl polymer or a derivative thereof, and an acrylic thickening agent. Of these, preferable is one or two or more selected from a carboxyvinyl polymer, a (dimethylacrylamide/sodium acryloyldimethyltaurate) crosspolymer, an (ammonium acryloyldimethyltaurate/VP) copolymer, an (ammonium acryloyldimethyltaurate/beheneth-25 methacrylate) crosspolymer, and a (sodium acrylate/sodium acryloyldimethyltaurate) copolymer.

(E) The polymer thickening agent can be contained in accordance with the usability required by an intended

formulation, preferably in an amount of 0.05 to 1.0% by mass based on the whole oil-in-water emulsion composition.

In the present invention, (F) a silicone elastomer may further be contained.

When a silicone elastomer is added to a composition such as a cosmetic, a user can experience a smooth gentle feel when used.

Examples of the silicone elastomer include a silicone elastomer (organopolysiloxane). The silicone elastomer includes, for example, a crosslinked silicone (crosslinked organopolysiloxane) in which silicone polymers are crosslinked three-dimensionally. A silicone elastomer, when used, can reduce stickiness and also achieve smoothness (a silky feel) on the skin when applied.

The silicone elastomer usable in the composition of the present application is not particularly limited as long as it is usable to the skin. Examples of the silicone elastomer include a dimethicone crosspolymer, a dimethicone/vinyl dimethicone crosspolymer, a dimethicone/phenyl vinyl dimethicone crosspolymer, a vinyl dimethicone/lauryl dimethicone crosspolymer, a lauryl polydimethylsiloxyethyl dimethicone/bis-vinyl dimethicone crosspolymer, an alkyl(C30-45)cetearyl dimethicone crosspolymer, and a cetearyl dimethicone crosspolymer.

A commercial product, for example, can be used as the silicone elastomer. A commercial product can be a mixture of the silicone elastomer and an oily component. The oily component comprised in a commercial product is not particularly limited as long as it is usable to the skin. Examples of the commercial product comprising the silicone elastomer include Gransil DMG-3 (Grant Industries, Inc.) comprising 12% by mass of polysilicone-11 as the silicone elastomer and 88% by mass of dimethicone as the oily component, and other products such as KSG-16 (Shin-Etsu Chemical Co., Ltd.), and Dow Corning (R) 9041 Silicone Elastomer Blend (Dow Corning Toray Co., Ltd.)

The content of the silicone elastomer in the composition of the present application is preferably 0.1% by mass or more, and more preferably 0.3% by mass or more, based on the gross amount of the composition.

In order to impart "smoothness when applied," which is demanded by purchasers as a cosmetic, glycerin may further be contained. When glycerin is contained in a large amount, typically stickiness is felt thereby failing to obtain good usability. In the present invention, even when glycerin is contained in a large amount, there is no stickiness and a smooth feel can be obtained.

The method for producing the oil-in-water emulsion composition according to the present invention has a vesicle formation step in which (A) the aqueous phase and (C) polyoxyalkylene-modified silicone are mixed to form vesicles. Further, a step can be added for adding an ionic surfactant to the vesicle-containing aqueous solution obtained in the above step.

In the method for producing the oil-in-water emulsion composition according to the present invention, (A) the aqueous phase and (C) polyoxyalkylene-modified silicone are mixed first to form a vesicle, which is the precursor of a nanodisc. (A) The aqueous phase herein is not particularly limited as long as it is a formula having water and an aqueous solvent (monohydric alcohol and/or dihydric glycol) as the main medium, and components typically used for cosmetics, other than water and the aqueous solvent, can be contained in a content not affecting the stability of nanodiscs.

(C) Polyoxyalkylene-modified silicone is dissolved in advance in the monohydric alcohol and/or the dihydric glycol, which is the constituting component of (A) the aqueous phase, and the resultant is mixed with water, which is the remaining constituting component of (A) the aqueous phase, whereby a vesicle, which is the precursor of a nanodisc composed of the polyoxyalkylene-modified silicone, is formed in the aqueous phase.

When an oil is added to and stirred with this aqueous phase comprising the vesicle, which is the precursor of a nanodisc, the vesicle, which is the precursor of a nanodisc, transitions to a nanodisc at the oil-water interface and is adsorbed thereto, whereby the present invention is accomplished.

The oil-in-water emulsion composition according to the present invention can be preferably used, for example, as a cosmetic. The composition, when used in a cosmetic, can contain components typically used for medicines and cosmetics other than the above essential components, in an amount not affecting the stability of the components. Further, an oil in an amount which cannot be contained by the typical solubilization can be contained in the present nanodisc-containing composition, while a refreshing feel with reduced stickiness can still be obtained when used.

Other formula components can be contained in the aqueous phase in advance before the vesicle formation, or can be contained in the formula after the vesicle formation.

The usage of the cosmetic according to the present invention is not particularly limited and can be preferably used as, for example, a toner, a skin care essence serum, an emulsion, a cream, a hair cream, a massage cream, a cleansing cream and the like.

## EXAMPLES

Hereinafter, the present invention will be described in further details in reference with examples of the present invention, but the present invention is not limited to these. The content, unless otherwise stated, is shown in % by mass below.

[Test Example 1] Creation of vesicles, the precursor of nanodiscs, and conditions for forming vesicles

The present inventors created aqueous phase parts shown in Table 1-1 and Table 1-2 by a usual method, and then mixed the aqueous phase with PEG-12 dimethicone to carry out a visual evaluation by specialists and measure an average particle size. The average particle size was measured using a zetasizer (Malvern Panalytical Ltd.'s zetasizer Nano ZS).

[Evaluation method]

A: When an average particle size at room temperature 25°C is 30 nm to 150 nm with a pale appearance, vesicles are decided to be present.
B: When an average particle size at room temperature 25°C is less than 30 nm with a colorless clear appearance, micelles are decided to be present.
C: When an average particle size at room temperature 25°C is more than 150 nm and less than 250 nm with a clouded appearance and aggregates being found, an insoluble state is decided.

[Table 1-1]

| Change in the particle by ratio of ethyl alcohol to the surfactant | | Amount of PEG-12 dimethicone (*1) | | | | |
|---|---|---|---|---|---|---|
| | | 0.5 | 1 | 1.5 | 2 | 5 |
| Amount of ethyl alcohol (% by mass) | 0 | C | C | C | C | C |
| | 2.5 | A | A | A | A | A |
| | 5 | A | A | A | A | A |
| | 10 | A | A | A | A | A |
| | 15 | A | A | A | A | A |
| | 20 | B | B | B | B | B |
| | 40 | B | B | B | B | B |
| (*1) DOWSIL ES-5373 (manufactured by Dow Toray Co., Ltd.) | | | | | | |

[Table 1-2]

| Change in the particle by ratio of dipropylene glycol to the surfactant | | Amount of PEG-12 dimethicone (*1) | | | | |
|---|---|---|---|---|---|---|
| | | 0.5 | 1 | 1.5 | 2 | 5 |
| Amount of dipropylene glycol (% by mass) | 0 | C | C | C | C | C |
| | 2.5 | A | A | A | A | A |
| | 5 | A | A | A | A | A |
| | 10 | A | A | A | A | A |
| | 15 | A | A | A | A | A |
| | 20 | A | A | A | A | A |
| | 40 | B | B | B | B | B |
| (*1) DOWSIL ES-5373 (manufactured by Dow Toray Co., Ltd.) | | | | | | |

As evident in Table 1-1 and Table 1-2, when the contents of ethyl alcohol and dipropylene glycol are less than 2.5% by mass, PEG-12 dimethicone does not dissolve in the aqueous phase. It is revealed that vesicles are formed when ethyl alcohol is 2.5% by mass to 15% by mass, and dipropylene glycol is 2.5% by mass to 20% by mass.

[Test Example 2]

Subsequently, the present inventors adjusted so that PEG-12 dimethicone was always 1.0% by mass in the composition, and studied on the relationship between the HLB difference of PEG-12 dimethicone and the content of ethyl alcohol. The evaluation method was the same as in Test Example 1. The results are shown in Table 2 and FIG. 1.

[Table 2]

| Change by ethyl alcohol and HLB of the surfactant | | Difference in the HLB of PEG-12 Dimethicone | | |
|---|---|---|---|---|
| | | HLB 5(*2) | HLB 8 (*1) | HLB 13(*3) |
| Amount of ethyl alcohol (% by mass) | 0 | C | C | B |
| | 5 | A | A | B |
| | 10 | A | A | B |
| (*1) DOWSIL ES-5373 (manufactured by Dow Toray Co., Ltd.)<br>(*2) DOWSIL SH 3775 M (manufactured by Dow Toray Co., Ltd.)<br>(*3) DOWSIL SH 3771 M (manufactured by Dow Toray Co., Ltd.) | | | | |

As evident in Table 2 and FIG. 1, vesicles, which are the precursor of nanodiscs, are formed when HLB of PEG-12 dimethicone is 5, and a content of ethyl alcohol is 5 to 50% by mass. Vesicles, which are the precursor of nanodiscs, are formed when HLB of PEG-12 dimethicone is 8, and a concentration of ethyl alcohol is 2.5 to 10% by mass. Further, vesicles, which are the precursor of nanodiscs, are not formed but micelles are formed when HLB of PEG-12 dimethicone is 13, regardless of the content of ethyl alcohol. Further, PEG-12 dimethicone does not dissolve in the aqueous phase when HLB of PEG-12 dimethicone is 7 or less, and a content of ethyl alcohol is 5% by mass or less.

FIG. 1 reveals that PEG-12 dimethicone having an HLB of 10 or less does not dissolve in water. PEG-12 dimethicone, which is not dissolved in water at this time, is incapable of emulsifying the oil, or is unstable with the passage of time. When an oil is emulsified in a composition capable of forming vesicles, which are the precursor of nanodiscs, in the presence of ethyl alcohol, the emulsification is enabled and stable.

[Test Example 3]

[Study on the oil amount for nanodisc emulsification]

Subsequently, the present inventors studied on the amount of oil when emulsifying using the vesicle, which is the precursor of nanodisc. The results are shown in Table 3.

The aqueous phase comprises only water, ethyl alcohol, and PEG-12 dimethicone, and the concentration of ethyl alcohol was set to be always 10% by mass in the aqueous phase. An oil was added to the aqueous phase. The concentration of PEG-12 dimethicone was set to be 1.0% by mass.

(Evaluation method for the states)

Evaluations were carried out as follows.

A: Appearance had creaming from immediately after the emulsification with the passage of time (4 weeks), but no notable coalescence or enlargement of emulsion particles was recognized under optical microscope observation.

B: Separation of the oil on appearance and coalescence of emulsion particles under optical microscope observation are found.

[Table 3]

| Text Example | | | 3 - 1 | 3 - 2 | 3 - 3 | 3 - 4 | 3 - 5 | 3 - 6 | 3 - 7 |
|---|---|---|---|---|---|---|---|---|---|
| Aqueous phase | Total aqueous phase | | 90 | 85 | 70 | 60 | 50 | 40 | 20 |
| | Water | | 80 | 75.5 | 62 | 53 | 44 | 35 | 17 |
| | Ethyl alcohol | | 9 | 8.5 | 7 | 6 | 5 | 4 | 2 |
| | PEG-12 Dimethicone (*1) | | 1 | 1 | 1 | 1 | 1 | 1 | 1 |
| Oil phase | Total oil phase | | 10 | 15 | 30 | 40 | 50 | 60 | 80 |
| | Evalua tion by the oil | Silicone oil alone (*4) | A | A | A | B | B | B | B |
| | | Hydrocarbon oil alone (*5) | A | A | A | A | A | A | B |
| | | Polar oil alone (*6) | A | A | A | A | A | A | B |
| | | Mixture of above 3 oils in 1: 1:1 | A | A | A | A | A | B | B |

(*1) DOWSIL ES-5373 (manufactured by Dow Toray Co., Ltd.)
(*4) Silicone KF-96A-6T (manufactured by Shin-Etsu Chemical Co., Ltd.)
(*5) NOMUCOAT HP-30 (manufactured by The Nisshin OilliO Group, LTD.)
(*6) RA-PE-408 (manufactured by NIPPOIN FINE CHEMICAL CO., LTD.)

Table 3 showed that the oil up to about 50% by mass can be stably contained. Further, when the oil was more than 60% by mass, the oil slightly floated, which is however not shown in the table. In the case of the silicone oil alone, it was shown that up to about 30% by mass was stably contained.

[Test Example 4]

Further, the present inventors studied on the molecular species of the oils. The results are shown in Table 4. The evaluation method is the same as in Test Example 3.

[Table 4]

| Text Example | | | 4 - 1 | 4 - 2 | 4 - 3 | 4 - 4 | 4 - 5 | 4 - 6 | 4 - 7 | 4 - 8 | 4 - 9 | 4 - 1 0 |
|---|---|---|---|---|---|---|---|---|---|---|---|---|
| Aqueous phase | Total aqueous phase | | 60 | 60 | 60 | 60 | 60 | 50 | 50 | 50 | 50 | 50 |
| | Water | | 53 | 53 | 53 | 53 | 53 | 44 | 44 | 44 | 44 | 44 |
| | Ethyl alcohol | | 6 | 6 | 6 | 6 | 6 | 5 | 5 | 5 | 5 | 5 |
| | PEG-12 Dimethicone (*1) | | 1 | 1 | 1 | 1 | 1 | 1 | 1 | 1 | 1 | 1 |
| Oil phase | Total oil phase | | 40 | 40 | 40 | 40 | 40 | 50 | 50 | 50 | 50 | 50 |
| | Ratio of silicone oil in oil (%) | | 90 | 70 | 50 | 30 | 10 | 90 | 70 | 50 | 30 | 10 |
| | Evaluation by the oil | Silicone oil(*4) : Hydrocarbon oil(*5) | B | B | A | A | A | B | B | A | A | A |
| | | Silicone oil(*4) : Polar oil(*6) | B | B | A | A | A | B | B | A | A | A |

(*1) DOWSIL ES-5373 (manufactured by Dow Toray Co., Ltd.)
(*4) Silicone KF-96A-6T (manufactured by Shin-Etsu Chemical Co., Ltd.)
(*5) NOMUCOAT HP-30 (manufactured by The Nisshin OilliO Group, LTD.)
(*6) RA-PE-408 (manufactured by NIPPOIN FINE CHEMICAL CO., LTD.)

Table 4 reveals that, in the case of 50% by mass or less of the oils in the content ratio of water to the oil, a content ratio of the silicone oil in the oil phase of more than 70% by mass finds coalescence of emulsion particles on

appearance and causes the separation of the oil. In a content ratio of the silicone oil in the oil phase of 50% by mass or less, appearance had creaming from immediately after the emulsification with the passage of time (4 weeks), but no coalescence or separation of the oil is found, and no notable coalescence or enlargement of emulsion particles is recognized under optical microscope observation, thereby revealing possibility of stable incorporation.

[Test Example 5] Study on emulsifying capacity by the oil molecular species

The present inventors studied on the difference in the emulsifying capacity by the molecular species of oils. The results are shown in Table 5. Evaluation was carried out as follows.

A: Change rate in the oil particle size under an optical microscope (particle size with the passage of time/initial particle size) is 0.8 to 1.2 of the initial value at a preservation temperature of 0°C to 50°C after 4 weeks has passed.
B: Above change rate is more than 1.2 or less than 0.8.

[Table 5]

| Test Example | | | 5 - 1 | 5 - 2 | 5 - 3 | 5 - 4 | 5 - 5 | 5 - 6 | 5 - 7 | 5 - 8 | 5 - 9 | 5 - 1 0 |
|---|---|---|---|---|---|---|---|---|---|---|---|---|
| Water | | Deionized water | Balance | Balance | Balance | Balance | Balance | Balance | Balance | Balance | Balance | Balance |
| Alcohol | | Ethyl alcohol | 8 | 8 | 8 | 8 | 8 | 8 | 8 | 8 | 8 | 8 |
| Active agents | | PEG-12 Dimethicone (*1) | 1 | 1 | 1 | 1 | 1 | 1 | 1 | 1 | 1 | 1 |
| | | Sodium N-stearoyl-N-methyltaurate | 0.01 | 0.01 | 0.01 | 0.01 | 0.01 | 0.01 | 0.01 | 0.01 | 0.01 | 0.01 |
| Oils | Silicone oils | Dimethicone(*4) | 40 | | | | | | | | | |
| | | Dimethicone(*7) | | 40 | | | | | | | | |
| | | Diphenyisitoxy phenyl trimethicone (*8) | | | 40 | | | | | | | |
| | Non-polar oils | Liquid paraffin | | | | 40 | | | | | | |
| | | Olefin oligomer | | | | | 40 | | | | | |
| | | Tetraisobutane | | | | | | 40 | | | | |
| | Polar oils | Pentaerythritol tetra(2-ethylhexanoate) | | | | | | | 40 | | | |
| | | Triethylhexanoin | | | | | | | | 40 | | |
| | | Cetyl 2-ethylhexanoate | | | | | | | | | 40 | |
| | | Tripropylene glycol | | | | | | | | | | 40 |
| Total | | | 100 | 100 | 100 | 100 | 100 | 100 | 100 | 100 | 100 | 100 |
| Evaluation | | | B | B | B | A | A | A | A | A | A | A |
| Test Example | | | 5 - 11 | 5 - 12 | 5 - 13 | 5 - 1 4 | 5 - 15 | 5 - 16 | 5 - 17 | 5-18 | 5 - 19 | 5 - 2 0 |
| Water | | Deionized water | Balance | Balance | Balance | Balance | Balance | Balance | Balance | Balance | Balance | Balance |
| Alcohol | | Ethyl alcohol | 8 | 8 | 8 | 8 | 8 | 8 | 8 | 8 | 8 | 8 |
| Active agents | | PEG-12 Dimethicone (*1) | 1 | 1 | 1 | 1 | 1 | 1 | 1 | 1 | 1 | 1 |
| | | Sodium N-stearoyl-N-methyltaurate | 0.01 | 0.01 | 0.01 | 0.01 | 0.01 | 0.01 | 0.01 | 0.01 | 0.01 | 0.01 |

(continued)

| Test Example | | | 5 - 11 | 5 - 12 | 5 - 13 | 5 - 1 4 | 5 - 15 | 5 - 16 | 5 - 17 | 5-18 | 5 - 19 | 5 - 2 0 |
|---|---|---|---|---|---|---|---|---|---|---|---|---|
| Oils | Silicone oils | Dimethicone(*4) | 50 | | | | | | | | | |
| | | Dimethicone(*7) | | 50 | | | | | | | | |
| | | Diphenylsiloxy phenyl trimethicone (*8) | | | 50 | | | | | | | |
| | Non-polar oils | Liquid paraffin | | | | 50 | | | | | | |
| | | Olefin oligomer | | | | | 50 | | | | | |
| | | Tetraisobutane | | | | | | 50 | | | | |
| | Polar oils | Pentaerythritol tetra(2-ethylhexanoate) | | | | | | | 50 | | | |
| | | Triethylhexanoin | | | | | | | | 50 | | |
| | | Cetyl 2-ethylhexanoate | | | | | | | | | 50 | |
| | | Tripropylene glycol | | | | | | | | | | 50 |
| Total | | | 100 | 100 | 100 | 100 | 100 | 100 | 100 | 100 | 100 | 100 |
| Evaluation | | | B | B | B | B | B | B | A | A | A | A |

(*1) DOWSIL ES-5373 (manufactured by Dow Toray Co., Ltd.)
(*4) Silicone KF-96A-6T (manufactured by Shin-Etsu Chemical Co., Ltd.)
(*7) KF-96L-1.5CS (manufactured by Shin-Etsu Chemical Co., Ltd.)
(*8) KF56A (manufactured by Shin-Etsu Chemical Co., Ltd.)

The results of Table 5 show that the oils that have high compatibility with PEG-12 dimethicone having an HLB of 10 or less and are difficult to be emulsified are in the order of silicone oil>hydrocarbon oil>polar oil. In other words, the oils having high emulsion stability can be in the order of polar oil>hydrocarbon oil>silicone oil. This shows that the emulsion stability tends to be poorer when an oil having high compatibility with a surfactant is in a large amount.

[Test Example 6]

The present inventors confirmed the emulsion state by the difference in alcohol (ethyl alcohol) content, and studied on the PEG-12 dimethicone structure before and after the emulsification.

Aqueous phases were prepared by the formulae as shown in Table 6, and then the oils were added thereto and treated using a homogenizer (7000 rpm, 3 minutes) to create emulsion compositions. The obtained emulsion composition was centrifuged (3000 rpm, 16 h), and the particle size peak value of the infranatant solution was measured using a Malvern Panalytical Ltd.'s zetasizer Nano ZS. FIG. 2, FIG. 4, and FIG. 6 show the results of the peak values of the aqueous phase before the emulsification, and FIG. 3, FIG. 5, and FIG. 7 show the results of the peak values after the emulsification and then centrifugation.

[Table 6]

| Test Example | | 6 - 1 | 6 - 2 | 6 - 3 |
|---|---|---|---|---|
| Aqueous phase | Deionized water | Balance | Balance | Balance |
| | Ethyl alcohol | 0 | 20 | 10 |
| | PEG-12 Dimethicone (HLB: 8) | 5 | 5 | 5 |
| Oils (1:1:1) | Dimethicone(*4) | 10 | 10 | 10 |
| | Olefin oligomer(*5) | | | |
| | Pentaerythrityl tetraethylhexanoate(*6) | | | |
| Total | | 100 | 100 | 100 |
| (*4) Silicone KF-96A-6T (manufactured by Shin-Etsu Chemical Co., Ltd.)<br>(*5) NOMUCOAT HP-30 (manufactured by The Nisshin OilliO Group, Ltd.)<br>(*6) RA-PE-408 (manufactured by NIPPON FINE CHEMICAL CO., LTD.) | | | | |

In Test Example 6, FIG. 2 and FIG. 3 show, in Test Example 6-1, that when an ethyl alcohol content is 0% by mass, PEG-12 dimethicone does not dissolve in the aqueous phase and is in a undissolved state. There is no significant difference found in the peak before and after the emulsification. In Test Example 6-2, FIG. 4 and FIG. 5 show that micelles are formed when an alcohol content is 20% by mass, and there is no significant difference found in the peak before and after the emulsification. Most of PEG-12 dimethicone are in a micelle state, thereby showing that the micelles are deformed, split thereby forming a monomolecular adsorption layer, and emulsified at the oil-water interface.

On the other hand, in Test Example 6-3, FIG. 6 and FIG. 7 show that when an ethyl alcohol content is 10% by mass, vesicles, which are the precursor of nanodiscs, are formed before the emulsification. A vesicle is typically smaller than 1 micron, which is the size of an emulsion particle, and larger than 10 nm, which is the size of a micelle. In the present system, particles having about 30 nm to 200 nm are formed, thereby revealing that these are vesicles. Further, Non Patent Literature 2 discloses the formation of vesicles in the present composition. It is considered that, after the emulsification, the emulsion is stabilized by the adsorption of nanodiscs, which is the deformed vesicle structure, at the oil-water interface.

[Test Example 7] Stability and texture in various states

Emulsion states were classified into undissolved (a state in which PEG-12 dimethicone is not dissolved in the aqueous phase), nanodisc (a state in which vesicles, which are the nanodisc precursor, are formed), and micelle (a state in which PEG-12 dimethicone is in the form of micelle in the aqueous phase), and the stability and texture of each state were studied.

For the undissolved, nanodisc, and micelle, aqueous phases were prepared using water, ethyl alcohol, and PEG-12 dimethicone by the formulae as shown in Table 7, and then the oils were added thereto and treated using a homogenizer (7000 rpm, 3 minutes) to create emulsion compositions.

An evaluation method for each item is shown below, and the results are shown in Table 7.

(Evaluation method for the centrifugal stability)

Changes in the emulsion particle size after centrifuged at 3,000 rpm for 16 hours, and 40,000 rpm for 1 hour, were observed using an optical microscope and evaluated.

A: No change was found in the emulsion particle size
B: Change was found in the emulsion particle size, but no change in the form such as separation was found
C: Separation was caused and the formulation was failed

(Evaluation method for the permeating feel)

Seven specialized panelists applied the present test products to the skin, evaluated the effects, and classified as follows based on the number of panelists who answered "there is a permeating feel to the skin."

A: 5 or more panelists
B: 3 to 4 panelists
C: 0 to 2 panelists

(Evaluation method for the spreadability when applied)

Seven specialized panelists applied the present test products to the skin, evaluated the effects as follows, and classified as follows based on the number of panelists who answered "there is spreadability on the skin."

A: 5 or more panelists
B :3 to 4 panelists
C: 0 to 2 panelists

(Evaluation method for the stickiness after applied)

Seven specialized panelists applied the present test products to the skin, evaluated the effects as follows, and classified as follows based on the number of panelists who answered "there is no stickiness."
A characteristic feel when used, if any, is noted as an aside

A: 5 or more panelists
B: 3 to 4 panelists
C: 0 to 2 panelists

[Table 7]

| Test Example | 7 - 1 | 7 - 2 | 7 - 3 |
|---|---|---|---|
| | Undissolved | Nanodisc | Micelle |
| Deionized water | Balance | Balance | Balance |
| Ethyl alcohol | 0 | 5 | 20 |
| PEG-12 Dimethicone (HLB: 8) | 5 | 5 | 5 |
| Oils (1:1:1) | 10 | 10 | 10 |
| Pentaerythrityl tetraethylhexanoate(*6) | | | |
| Olefin oligomer(*5) | | | |
| Dimethicone(*4) | | | |
| Total | 100 | 100 | 100 |

(continued)

| Test Example | | 7 - 1 | 7 - 2 | 7 - 3 |
|---|---|---|---|---|
| | | Undissolved | Nanodisc | Micelle |
| Evaluations | Stability after centrifugation | B | A | B |
| | Permeating feel | B | A | C |
| | Spreadability when applied | C | A | B |
| | Stickiness after applied | C | A | B |
| (*4) Silicone KF-96A-6T (manufactured by Shin-Etsu Chemical Co., Ltd.)<br>(*5) NOMUCOAT HP-30 (manufactured by The Nisshin OilliO Group, Ltd.)<br>(*6) RA-PE-408 (manufactured by NIPPON FINE CHEMICAL CO., LTD.) | | | | |

In Test Example 7, the results in Table 7 show that the state in which nanodiscs are formed as shown in Test Example 7-2 had better results in both stability and texture than other states.

[Test Example 8]

The nanodisc emulsion composition according to the present invention is preferably contained in a cosmetic.
The present inventors studied on the content when PEG-12 dimethicone is added to a cosmetic. The results are shown in Table 8. Evaluation methods for the appearance and texture were carried out as follows.

(Evaluation method for the states)

Evaluations were carried out as follows.

A: Appearance had creaming from immediately after the emulsification with the passage of time (4 weeks), but no notable coalescence or enlargement of emulsion particles was recognized under optical microscope observation.
B: Separation of the oil on appearance and coalescence of emulsion particles under optical microscope observation are found.

(Evaluation method for the spreadability when applied)

Seven specialized panelists applied the present test products to the skin, evaluated the effects as follows, and classified as follows based on the number of panelists who answered "the formulation spreads smoothly without causing fingers to stop during application."

A: 5 or more panelists
B: 3 to 4 panelists
C: 0 to 2 panelists

(Evaluation method for the sticky feel after applied)

Seven specialized panelists applied the present test products to the skin, evaluated the effects as follows, and classified as follows based on the number of panelists who answered "there is no stickiness."

A: 5 or more panelists
B: 3 to 4 panelists
C: 0 to 2 panelists

[Table 8]

| Test Example | 8 - 1 | 8 - 2 | 8 - 3 | 8 - 4 |
|---|---|---|---|---|
| PEG-12 Dimethicone (HLB: 8) | 0.4 | 0.6 | 0.8 | 1 |

(continued)

| Test Example | | 8 - 1 | 8 - 2 | 8 - 3 | 8 - 4 |
|---|---|---|---|---|---|
| Ethyl alcohol | | 5 | 5 | 5 | 5 |
| Dipropylene glycol | | 6.5 | 6.5 | 6.5 | 6.5 |
| Deionized water | | Balance | Balance | Balance | Balance |
| Oils | Olefin oligomer(*5) | 2 | 2 | 2 | 2 |
| | Vaseline | 2 | 2 | 2 | 2 |
| | Dimethicone(*7) | 1 | 1 | 1 | 1 |
| | Dimethicone(*8) | 1 | 1 | 1 | 1 |
| | Pentaerythrityl tetraethylhexaricate(*6) | 2 | 2 | 2 | 2 |
| | Di(pliytosteryl/2-octyldodecyl) N-lauroyl-L-glutamate(*9) | 0.01 | 0.01 | 0,01 | 0.01 |
| Total | | 100 | 100 | 100 | 100 |
| Evaluations | Appearance | A | A | A | A |
| | Spreadability when applied | C | B | A | A |
| | Stickiness after applied | C | B | A | A |
| (*5) NOMUCOAT HP-30 (manufactured by The Nisshin OilliO Group, Ltd.)<br>(*6) RA-PE-408 (manufactured by NIPPON FINE CHEMICAL CO., LTD.)<br>(*7) KF-96L-1.5CS (manufactured by Shin-Etsu Chemical Co., Ltd.)<br>(*8) KF56A (manufactured by Shin-Etsu Chemical Co., Ltd.)<br>(*9) ELDEW PS-203R (manufactured by Ajinomoto Co., Inc.) | | | | | |

Table 8 showed that when a content of PEG-12 dimethicone is more than 0.6% by mass, the cosmetics had a good feel when used. It was also shown that when PEG-12 dimethicone is more than 0.8% by mass, a feel when used is much better.

[Test Example 9]

The present inventors studied on the content when an ionic surfactant is added to a cosmetic. The results are shown in Table 9.

For confirming the stability, after preservation at 50°C for 1 week, the state of emulsion particles was observed using an optical microscope.

(Evaluation for the change in the emulsion particle size)

A: No change was found in the emulsion particle size
B: Change was found in the emulsion particle size, but no change in the form such as separation was found
C: Separation was caused and the formulation was failed

[Table 9]

| Test Example | 9 - 1 | 9 - 2 | 9 - 3 | 9 - 4 | 9 - 5 | 9 - 6 |
|---|---|---|---|---|---|---|
| Deionized water | Balance | Balance | Balance | Balance | Balance | Balance |
| Ethyl alcohol | 5 | 5 | 5 | 5 | 5 | 5 |
| Glycerin | 7 | 7 | 7 | 7 | 7 | 7 |
| Dipropylene glycol | 3.5 | 3.5 | 3.5 | 3.5 | 3.5 | 3.5 |

(continued)

| Test Example | | 9 - 1 | 9 - 2 | 9 - 3 | 9 - 4 | 9 - 5 | 9 - 6 |
|---|---|---|---|---|---|---|---|
| 1,3-Butylene glycol | | 3.5 | 3.5 | 3.5 | 3.5 | 3.5 | 3.5 |
| PEG-12 Dimethicone (HLB: 8) | | 1 | 1 | 1 | 1 | 1 | 1 |
| Sodium N-stearoyl-N-methyltaurate | | 0.01 | 0.02 | 0.04 | 0.06 | 0.08 | 0.1 |
| Tripropylene glycol dipivalate | | 2 | 2 | 2 | 2 | 2 | 2 |
| Diisopropyl Sebacate | | 3 | 3 | 3 | 3 | 3 | 3 |
| Dimethicone(*7) | | 2 | 2 | 2 | 2 | 2 | 2 |
| Diphenylsiloxy phenyl trimethicone(*8) | | 2 | 2 | 2 | 2 | 2 | 2 |
| Olefin oligomer | | 4 | 4 | 4 | 4 | 4 | 4 |
| Retinol | | q.s. | q.s. | q.s. | q.s. | q.s. | q.s. |
| Sodium acetylated hyaluronate | | q.s. | q.s. | q.s. | q.s. | q.s. | q.s. |
| Turmeric extract | | q.s. | q.s. | q.s. | q.s. | q.s. | q.s. |
| Total | | 100 | 100 | 100 | 100 | 100 | 100 |
| Evaluation | Stability | A | A | A | A | A | A |
| | Emulsion particles (μm) | 1 ~ 3 | 1 ~ 3 | 1 ~ 3 | 1 ~ 3 | 1 ~ 2.5 | 1 ~ 2.5 |
| (*5) NOMUCOAT HP-30 (manufactured by The Nisshin OilliO Group, Ltd.)<br>(*7) KF-96L-1.5CS (manufactured by Shin-Etsu Chemical Co., Ltd.)<br>(*8) KF56A (manufactured by Shin-Etsu Chemical Co., Ltd.) | | | | | | | |

Table 9 showed that the stability of the formulations was good when the ionic surfactant was contained in amounts of 0.01 to 0.1% by mass.

[Test Example 10]

The present inventors studied on the effect when polyoxyalkylene-modified silicone and an anionic surfactant are combined.

The texture and stability were evaluated as follows. The emulsion particle size was observed using an optical microscope. The results are shown in Table 10.

(Evaluation criteria on the texture)

A: 9 to 10 out of 10 specialized panelists evaluated as not sticky
B: 7 to 8 out of 10 specialized panelists evaluated as not sticky
C: 4 to 6 out of 10 specialized panelists evaluated as not sticky
D: 3 or less out of 10 specialized panelists evaluated as not sticky

(Evaluation criteria on the stability)

A: Average particle size after preservation at 50°C for 2 weeks has no change at all from the particle size immediately after prepared
B: Average particle size after preservation at 50°C for 2 weeks is less than 1.1 of the particle size immediately after prepared
C: Average particle size after preservation at 50°C for 2 weeks is 1.1 or more and less than 1.5 of the particle size immediately after prepared

D: Average particle size after preservation at 50°C for 2 weeks is 1.5 or more of the particle size immediately after prepared

[Table 10]

| Test Example | | 10 - 1 | 10 - 2 | 10 - 3 | 10 - 4 |
|---|---|---|---|---|---|
| Deionized water | | Balance | Balance | Balance | Balance |
| PEG-12 Dimethicone (HLB: 8) | | 1 | 1 | 1 | 1 |
| Ethyl alcohol | | 5 | 5 | 5 | 5 |
| 1,3-Butylene glycol | | 5 | 5 | 5 | 5 |
| Squalane | | 15 | 15 | 15 | 15 |
| Sodium N-stearoyl-N-methyltaurate | | | 0.01 | | 0.6 |
| disodium N-stearoyl-L -glutamate(*9) | | | | 0.6 | |
| Total | | 100 | 100 | 100 | 100 |
| Evaluation | Stickiness after applied | A | A | C | A |
| | Stability | B | A | C | B |
| (*9)Amisoft HS21(manufactured by Ajinomoto Co., Inc.) | | | | | |

As evident in Table 10, in the evaluation after 2 weeks from the sample preparation, it is essential for an anionic surfactant to have a content or conditions which do not inhibit the nanodisc formation. In the case of Sodium N-stearoyl-N-methyltaurate, good texture and stability are maintained even in 0.6% by mass, whereas disodium N-stearoyl-L-glutamate failed to obtain the effects in both texture and stability. It is suggested that Sodium N-stearoyl-N-methyltaurate does not affect the nanodisc formation. On the other hand, it is suggested that disodium N-stearoyl-L-glutamate inhibits the nanodisc formation. It is shown that Sodium N-stearoyl-N-methyltaurate in a content of 0.01% by mass has more excellent effect in the stability than 0.6% by mass.

[Test Example 11]

The present inventors studied on the content when a thickening agent is added to a cosmetic. Evaluation methods for appearance and texture are shown below, and the results are shown in Table 11.

(Evaluation method for the states)

A: Appearance has no creaming and the like from immediately after the emulsification with the passage of time (4 weeks).
B: Changes in the state such as creaming are found with the passage of time (4 weeks).

(Evaluation method for the refreshing feel when applied)

Seven specialized panelists applied the present test products to the skin, evaluated the effects as follows, and classified as follows based on the number of panelists who answered "there was a refreshing feel."

A: 5 or more panelists
B: 3 to 4 panelists
C: 0 to 2 panelists

[Table 11]

| Test Example | | 11 - 1 | 11 - 2 | 11 - 3 | 11 - 4 | 11 - 5 |
|---|---|---|---|---|---|---|
| Deionized water | | Balance | Balance | Balance | Balance | Balance |
| Ethyl alcohol | | 4 | 4 | 4 | 4 | 4 |
| Glycerin | | 3 | 3 | 3 | 3 | 3 |
| Dipropylene glycol | | 5 | 5 | 5 | 5 | 5 |
| 1.3-Butylene glycol | | 7 | 7 | 7 | 7 | 7 |
| Polyethylene glycol | | 3 | 3 | 3 | 3 | 3 |
| (Sodium acrylate/sodium acryloyidimethyltaurate) copolymer | | 0,9 | | | | |
| Hydroxyethyl cellulose | | 0.3 | | | | |
| (Dimethylacrylamide/sodium acryloyldimethyltaurate) crosspolymer | | | 0.8 | | | |
| Alkyl acrylate-alkyl methacrylate-polyoxyethylene stearyl ether methacrylate (20E.O.) copolymer emulsion | | | | 0.75 | | |
| Carboxyvinyl polymer | | | | | 0.3 | |
| Xanthan gum | | | | | | 0.1 |
| Polyvinyl alcohol | | 0.3 | 0.3 | 0.3 | 0.3 | 0.3 |
| Sodium N-stearoyl-N-methyltaurate | | 0.01 | 0.01 | 0.01 | 0.01 | 0.01 |
| PEG-12 Dimethicone (HLB: 8) | | 1 | 1 | 1 | 1 | 1 |
| Dimethicone(*4) | | 5 | 5 | 5 | 5 | 5 |
| Dimethicone(*7) | | 5 | 5 | 5 | 5 | 5 |
| Tripropylene glycol dipivalate | | 5 | 5 | 5 | 5 | 5 |
| Diisopropyl sebacate | | 10 | 10 | 10 | 10 | 10 |
| Isohexadecane | | 0.6 | | | | |
| Retinol | | q.s. | q.s. | q.s. | q.s. | q.s. |
| Potassium hydroxide | | | | | q.s. | |
| Polysorbate 80 | | q.s. | | | | |
| Sorbitan oleate | | q.s. | | | | |
| Dibutylated hydroxytoluene | | q.s. | q.s. | q.s. | q.s. | q.s. |
| Sodium metabisulfite | | q.s. | q.s. | q.s. | q.s. | q.s. |
| EDTA - 3Na. 2H2O | | q.s. | q.s. | q.s. | q.s. | q.s. |
| Phenoxy ethanol | | q.s. | q.s. | q.s. | q.s. | q.s. |
| Perfume | | q.s. | q.s. | q.s. | q.s. | q.s. |
| Total | | 100 | 100 | 100 | 100 | 100 |
| Evaluations | Appearance | A | A | A | A | B |
| | Feel when used (Refreshing feel) | A | A | A | A | |
| (*4) Silicone KF-96A-6T (manufactured by Shin-Etsu Chemical Co., Ltd.)<br>(*7) KF-96L-1.5CS (manufactured by Shin-Etsu Chemical Co., Ltd.) | | | | | | |

As shown in Table 11, it was shown that a refreshing feel can be obtained regardless of the kind of thickening agent in Test Examples which did not cause creaming.

[Test Example 12]

The present inventors studied on a feel when used when glycerin is contained in a large amount as a moisturizer in the nanodisc emulsion cosmetic. The results are shown in Table 12. A sticky feel after applied was evaluated in the same manner as in Test Example 7. The smoothness is the result obtained when compared with Test Example 12-1.

For Test Examples, PEG-12 dimethicone dissolved in ethyl alcohol was added to the aqueous phase containing water and glycerin to form vesicles, which are to be the nanodisc precursor. An oil phase was added to this aqueous phase to prepare an emulsion composition. However, glycerin can be added after PEG-12 dimethicone dissolved in ethyl alcohol was added to the aqueous phase containing water.

[Table 12]

| Test Example | | 12 - 1 | 12 - 2 |
|---|---|---|---|
| Deionized water | | Balance | Balance |
| Ethyl alcohol | | 4 | 4 |
| Glycerin | | 3 | 15 |
| Dipropylene glycol | | 5 | 5 |
| 1.3-Butylene glycol | | 7 | 7 |
| Polyethylene glycol | | 3 | 3 |
| (Sodium acrylate/sodium acryloyldimethyltaurate) copolymer | | 0.9 | 0.9 |
| Hydroxyethyl cellulose | | 0.3 | 0.3 |
| Polyvinyl alcohol | | 0.3 | 0.3 |
| Sodium N-stearoyl-N-methyltaurate | | 0.01 | 0.01 |
| PEG-12 Dimethicone (HLB: 8) | | 1 | 1 |
| Dimethicone(*4) | | 5 | 5 |
| Dimethicone(*7) | | 5 | 5 |
| Tripropylene glycol dipivalate | | 5 | 5 |
| Diisopropyl sebacate | | 10 | 10 |
| Isohexadecane | | 0.6 | 0.6 |
| Retinol | | q.s. | q.s. |
| Dibutylated hydroxytoluene | | q.s. | q.s. |
| Sodium metabisulfite | | q.s. | q.s. |
| EDTA- 3 Na.2H2O | | q.s. | q.s. |
| Phenoxy ethanol | | q.s. | q.s. |
| Perfume | | q.s. | q.s. |
| Total | | 100 | 100 |
| Evaluations | Stickiness after applied | A | A |
| | Smoothness | | Smoother than Test Example 12-1 |
| (*4) Silicone KF-96A-6T (manufactured by Shin-Etsu Chemical Co., Ltd.)<br>(*7) KF-96L-1.5CS (manufactured by Shin-Etsu Chemical Co., Ltd.) | | | |

Table 12 revealed that the smoothness is enhanced without the stickiness distinctive to glycerin.

When glycerin was used as a moisturizer in the cosmetics, the case of a micelle state posed a problem in the

texture because hydrate crystals and micelles remain as water volatilizes. However, in the nanodisc emulsion cosmetic, lamellar liquid crystals of a low viscosity are generated thereby enhancing the smoothness by glycerin.

[Test Example 13] Effects of the oil-in-water emulsion cosmetic containing an elastomer

The oil-in-water emulsion composition according to the present invention can also comprise (F) an elastomer. The present inventors studied on the texture when elastomers were contained as shown in Table 13. The sticky feel was confirmed by specialized panelists in the same manner as in Test Example 7.

[Table 13]

| Test Example | | 13 - 1 | 13 - 2 | 13 - 3 | 13 - 4 |
|---|---|---|---|---|---|
| Deionized water | | Balance | Balance | Balance | Balance |
| Ethyl alcohol | | 5 | 8 | 8 | 8 |
| Glycerin | | 15 | 15 | 15 | 15 |
| Dipropylene glycol | | 5 | 5 | 5 | 5 |
| 1,3-Butylene glycol | | 5 | 5 | 5 | 5 |
| Carbomer Na | | 1 | 1 | 1 | 1 |
| Polyvinyl alcohol | | 0.3 | 0.3 | 0.3 | 0.3 |
| Sodium N-stearoyl-N-methyltaurate | | 0.01 | 0.01 | 0.01 | 0.01 |
| PEG-12 Dimethicone (HLB: 8) | | 1 | 1 | 1 | 1 |
| Dimethicone(*4) | | 5 | 6.3 | 4.6 | 2.5 |
| Dimethicone(*7) | | 5 | 2.5 | 5 | 2.5 |
| Diphenylsiloxy phenyl trimethicone | | | | | 4.2 |
| Tripropylene glycol dipivalate | | 5 | 5 | 5 | 5 |
| Diisopropyl sebacate | | 10 | 10 | 10 | 10 |
| (Dimethicone/vinyl dimethicone) cross polymer | | | 1.2 | | |
| (Dimethicone/PEG-10/15) crosspolymer | | | | 0.36 | |
| (Dimethicone/phenyl vinyl dimethicone) crosspolymer | | | | | 0.8 |
| Retinol | | q.s. | q.s. | q.s. | q.s. |
| Dibutylated hydroxytoluene | | q.s. | q.s. | q.s. | q.s. |
| Sodium metabisulfite | | q.s. | q.s. | q.s. | q.s. |
| EDTA- 3 Na.2H2O | | q.s. | q.s. | q.s. | q.s. |
| Phenoxy ethanol | | q.s. | q.s. | q.s. | q.s. |
| Perfume | | q.S. | q.s. | q.s. | q.s. |
| Total | | 100 | 100 | 100 | 100 |
| Evaluation | Stickiness after applied | B | A | A | A |
| (*4) Silicone KF-96A-6T (manufactured by Shin-Etsu Chemical Co., Ltd.)<br>(*7) KF-96L-1.5CS (manufactured by Shin-Etsu Chemical Co., Ltd.)<br>(*8) KF56A (manufactured by Shin-Etsu Chemical Co., Ltd.) | | | | | |

Table 13 showed that without a sticky feel can further be obtained when elastomers were contained than when elastomers were not contained.

[Test Example 14]

[Freeze-fracture replication transmission electron microscopy (FF-TEM) observation]

FIG. 8 and FIG. 9 present freeze-fracture replication transmission electron microscopy photographs of the nanodisc emulsion obtained by the formula below. The freeze-fracture replication transmission electron microscope was carried out using Hitachi's H-8600. Freeze replication creation was carried out by Hitachi's BAF 400. A frozen sample was fractured under conditions at high vacuum and -140°C or less, and platinum and carbon were deposited on the sample at a 45° angle.

<Formula>

Aqueous phase

| | |
|---|---|
| Water | Balance |
| Ethyl alcohol | 2% by mass |
| PEG-12 Dimethicone (HLB: 8) | 1% by mass |

Oil phase

| | | |
|---|---|---|
| Silicone oil (*4) | 3% by mass | |
| Hydrocarbon oil (*5) | | 3% by mass |
| Polar oil (*6) | 3% by mass | |

(*4) Silicone KF-96A-6T (manufactured by Shin-Etsu Chemical Co., Ltd.)
(*5) NOMUCOAT HP-30 (manufactured by The Nisshin OilliO Group, Ltd.)
(*6) RA-PE-408 (manufactured by NIPPON FINE CHEMICAL CO., LTD.)

FIG. 8 and FIG. 9 show that oval shape nanodiscs surround the surface of an oil drop. FIG. 10 shows a schematic drawing of the photographs shown in FIG. 8 and FIG. 9.

Formulation Example 1: Cream

| (Formula) | (% by mass) |
|---|---|
| Deionized water | Balance |
| Ethyl alcohol | 5 |
| Glycerin | 10 |
| 1,3-Butylene glycol | 5 |
| Dipropylene glycol | 3 |
| Xanthan gum | 0.07 |
| (Sodium acrylate/sodium acryloyldimethyltaurate) copolymer | 0.9 |
| Isohexadecane | 0.6 |
| Polysorbate 80 | 0.2 |
| Sorbitan oleate | 0.06 |
| Sodium N-stearoyl-N-methyltaurate | 0.01 |
| PEG-12 Dimethicone (HLB 8) | 1 |
| Pentaerythritol tetra 2-ethylhexanoate | 12 |
| Hydrogenated polydecene | 5 |
| Methyl polysiloxane | 2 |

(continued)

| (Formula) | (% by mass) |
|---|---|
| Tripropylene glycol dipivalate | 1 |
| Retinol | q.s. |
| Tocopherol acetate | 0.1 |
| BHT | q.s. |
| Trisodium EDTA | q.s. |
| Phenoxy ethanol | q.s. |

Formulation Example 2: Essence serum

| (Formula) | (% by mass) |
|---|---|
| Deionized water | Balance |
| Ethyl alcohol | 5 |
| Glycerin | 5 |
| 1,3-Butylene glycol | 4 |
| PEG/PPG-14/7 Dimethyl ether | 1 |
| Xanthan gum | 0.05 |
| Carbomer | 0.45 |
| Potassium hydroxide | 0.2 |
| Sodium N-stearoyl-N-methyltaurate | 0.01 |
| PEG-12 Dimethicone (HLB 8) | 1 |
| Triethylhexanoin | 5 |
| Cetyl ethylhexanoate | 2 |
| Isododecane | 3 |
| Methyl polysiloxane | 4 |
| Tranexamic acid | 1 |
| Dipotassium glycyrrhizinate | 0.1 |
| Sodium metabisulfite | q.s |
| Trisodium EDTA | q.s |
| Phenoxy ethanol | q.s |
| Perfume | q.s |

Formulation Example 3: Essence serum

| (Formulation Example) | (% by mass) |
|---|---|
| Deionized water | Balance |
| Ethyl alcohol | 5 |
| Glycerin | 15 |
| 1,3-Butylene glycol | 10 |
| Xanthan gum | 0.05 |
| (Acrylates/steareth-20 methacrylate) copolymer | 0.6 |
| Sodium lauryl sulfate | 0.003 |
| Caustic potash | 0.1 |
| Sodium N-stearoyl-N-methyltaurate | 0.01 |
| PEG-12 Dimethicone (HLB 8) | 1.5 |
| Glyceryl diisostearate | 5 |
| Diisostearyl malate | 3 |

(continued)

| (Formulation Example) | (% by mass) |
|---|---|
| (Caprylic/capric) triglyceride | 3 |
| Isohexadecane | 2 |
| Diphenylsiloxy phenyl trimethicone | 1 |
| Nicotinamide | 5 |
| Trisodium EDTA | q.s. |
| Phenoxy ethanol | q.s. |
| Perfume | q.s. |

Formulation Example 4: Essence serum

| (Formula) | (% by mass) |
|---|---|
| Deionized water | Balance |
| Ethyl alcohol | 5 |
| Glycerin | 15 |
| 1,3-Butylene glycol | 5 |
| Xylitol | 1 |
| (Dimethylacrylamide/sodium acryloyldimethyltaurate) crosspolymer | 0.8 |
| Sodium N-stearoyl-N-methyltaurate | 0.01 |
| PEG-12 Dimethicone (HLB 8) | 1.2 |
| Meadowfoam oil | 5 |
| methyl polysiloxane | 5 |
| Tripropylene glycol dipivalate | 5 |
| Diisopropyl sebacate | 7 |
| Vitamin-A Acetate | 0.2 |
| Vitamin-E Acetate | 0.1 |
| BHT | q.s. |
| Trisodium EDTA | q.s. |
| Methyl paraben | q.s. |
| Phenoxy ethanol | q.s. |
| Perfume | q.s. |

Formulation Example 5: Essence serum

| (Formula) | (% by mass) |
|---|---|
| Deionized water | Balance |
| Ethyl alcohol | 10 |
| Glycerin | 3 |
| 1,3-Butylene glycol | 2 |
| Dipropylene glycol | 2 |
| Erythritol | 1 |
| Succinoglycan | 0.5 |
| Agar | 0.4 |
| Sodium N-stearoyl-N-methyltaurate | 0.01 |
| PEG-12 Dimethicone (HLB 5) | 1 |
| Glyceryl diisostearate | 3 |
| Diisostearyl malate | 2 |

(continued)

| (Formula) | (% by mass) | |
|---|---|---|
| (Caprylic/capric) triglyceride | 2 | |
| Squalane | | 1 |
| Methyl polysiloxane | | 2 |
| Di(phytosteryl/octyldodecyl) lauroyl glutamate | | 1 |
| 4-Methoxysalicylic acid potassium salt | 1 | |
| 2-O-Ethyl Ascorbic Acid | | 0.1 |
| Sodium metabisulfite | | q.s |
| Disodium EDTA | | q.s |
| Methyl paraben | | q.s |
| Phenoxy ethanol | | q.s |
| Perfume | | q.s |

**Claims**

1. An oil-in-water emulsion composition comprising (A) an aqueous phase, (B) an oil phase, and (C) polyoxyalkylene-modified silicone,
wherein the oil-in-water emulsion composition comprises:

    1 to 35% by mass in total of a monohydric alcohol and a dihydric glycol in (A) the aqueous phase, with the monohydric alcohol alone in a range of 1 to 15% by mass, and the dihydric glycol alone in a range of 1 to 20% by mass;
    1 to 50% by mass of (B) the oil phase; and
    0.2 to 5% by mass of (C) based on the whole composition.

2. The oil-in-water emulsion composition according to claim 1, wherein a content of the component (C) is 0.2 to 2.5% by mass.

3. The oil-in-water emulsion composition according to claim 1 or 2, wherein a proportion of a silicone oil in (B) the oil phase is 50% by mass or less.

4. The oil-in-water emulsion composition according to claims 1 to 3, wherein the component (C) is PEG-12 dimethicone.

5. The oil-in-water emulsion composition according to claims 1 to 4, wherein PEG-12 dimethicone in a concentration of 5 to 20% by mass does not dissolve but precipitates in water.

6. The oil-in-water emulsion composition according to claims 1 to 5, wherein PEG-12 dimethicone has an HLB of less than 10 as calculated by Griffin's formula.

7. The oil-in-water emulsion composition according to claims 1 to 6, wherein, when the emulsion composition is centrifuged at 40000 rpm for 60 minutes, particles having an average particle size of 30 nm to 150 nm are present in a clear layer separated to a lower layer.

8. The oil-in-water emulsion composition according to claims 1 to 7, wherein, when the emulsion composition is centrifuged at 3000 rpm for 16 hours, a clear separated layer of an oil at a proportion of 2% in the total volume is not observed in an upper layer or the lower layer.

9. The oil-in-water emulsion composition according to claims 1 to 8, wherein a lamellar nanodisc is adsorbed to an oil-water interface.

10. The oil-in-water emulsion composition according to claims 1 to 9, wherein a major axis of the nanodisc ranges from 20 nm to 1000 nm.

11. The oil-in-water emulsion composition according to claim 1, comprising, as a component (D), one or two or more

ionic surfactants selected from a sulfosuccinic acid diester salt, an alkyl aryl sulfonic acid salt, an alkyl ether sulfonic acid salt, a sulfosuccinic acid ester salt, an acyl methyltaurine salt, and an acyl taurine salt.

**12.** The oil-in-water emulsion composition according to claim 11, wherein the ionic surfactant is an N-stearoyl-N-methyltaurine salt.

**13.** The oil-in-water emulsion composition according to claim 11 or 12, wherein a content of (D) the ionic surfactant is 0.01 to 1.0% by mass based on the whole oil-in-water emulsion composition.

**14.** The oil-in-water emulsion composition according to claim 1, further comprising, as a component (E), a polymer thickening agent in a concentration of 0.05 to 1.0% by mass.

**15.** The oil-in-water emulsion composition according to claim 14, wherein the comprised polymer thickening agent is a carboxyvinyl polymer or a derivative thereof.

**16.** The oil-in-water emulsion composition according to claim 15, wherein the comprised polymer thickening agent is an acrylic thickening agent.

**17.** The oil-in-water emulsion composition according to claim 16, wherein the acrylic thickening agent is one or two or more acrylic thickening agents selected from a (dimethylacrylamide/sodium acryloyldimethyltaurate) crosspolymer, an ammonium acryloyldimethyltaurate/VP copolymer, an (ammonium acryloyldimethyltaurate/beheneth-25 methacrylate) crosspolymer, and a (sodium acrylate/sodium acryloyldimethyltaurate) copolymer.

**18.** The oil-in-water emulsion composition according to claim 1, comprising (F) an elastomer.

**19.** The oil-in-water emulsion composition according to claim 1, wherein the monohydric alcohol is ethyl alcohol.

**20.** The oil-in-water emulsion composition according to claim 1, wherein the dihydric glycol is dipropylene glycol.

[FIG. 1]

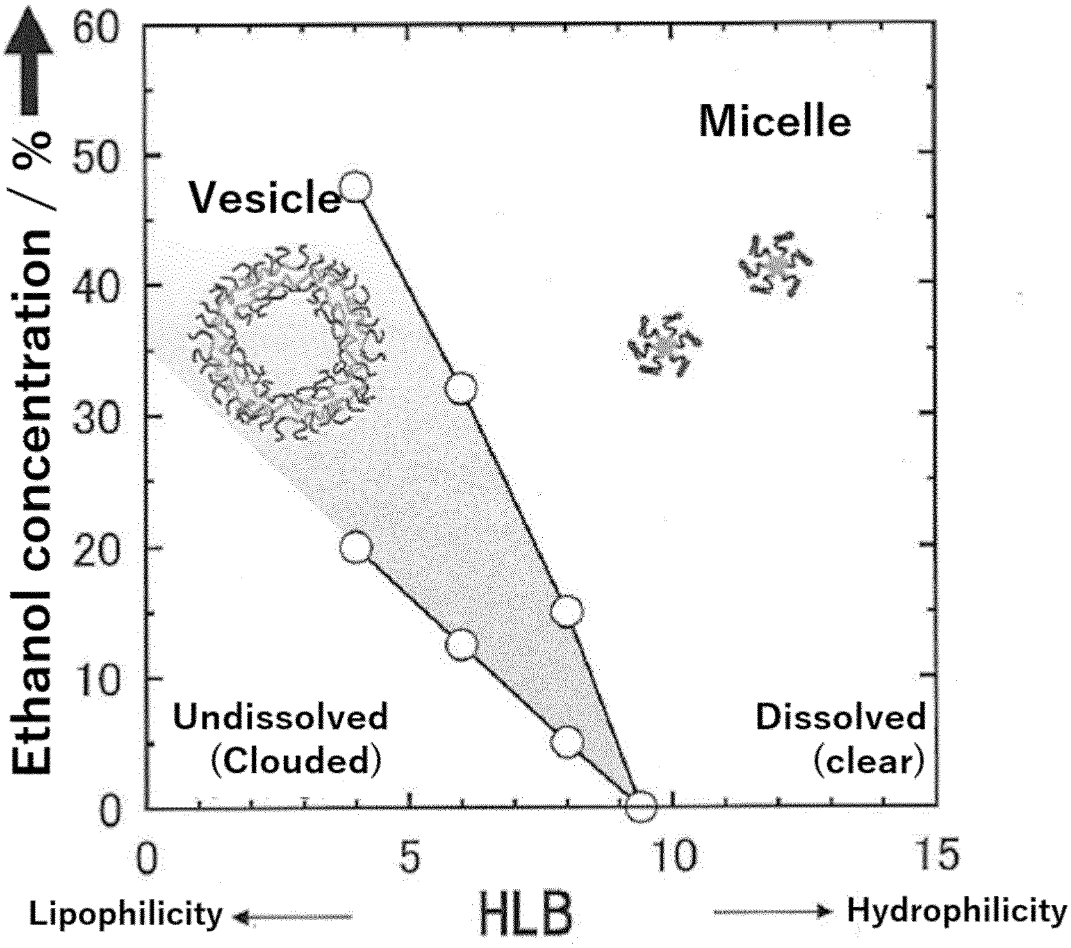
Micelle
Vesicle
Ethanol concentration / %
60
50
40
30
20
10
0
Undissolved
(Clouded)
Dissolved
(clear)
0
5
10
15
Lipophilicity
HLB
Hydrophilicity

[FIG. 2]

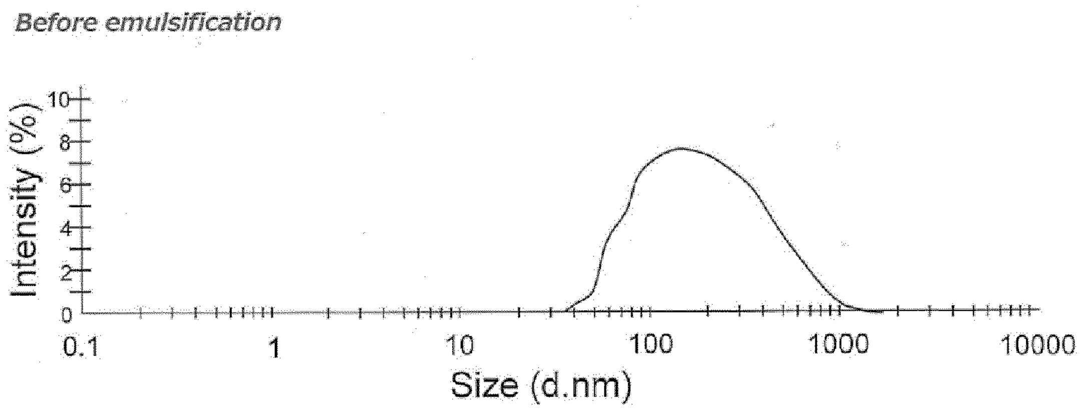
Before emulsification
Intensity (%)
10
8
6
4
2
0
0.1
1
10
100
1000
10000
Size (d.nm)

[FIG. 3]

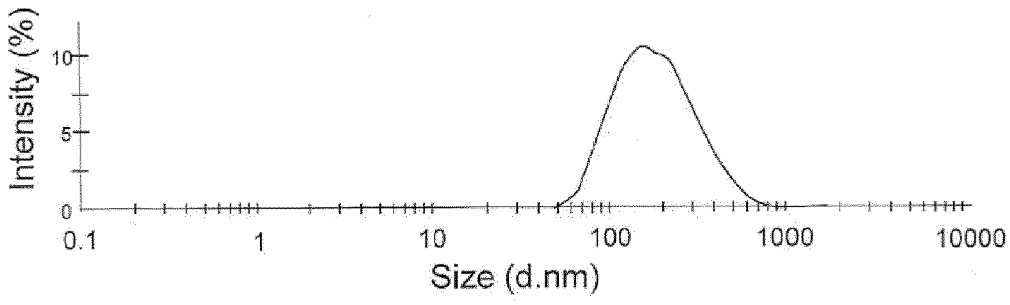


After centrifuge

[FIG. 4]

Before emulsification

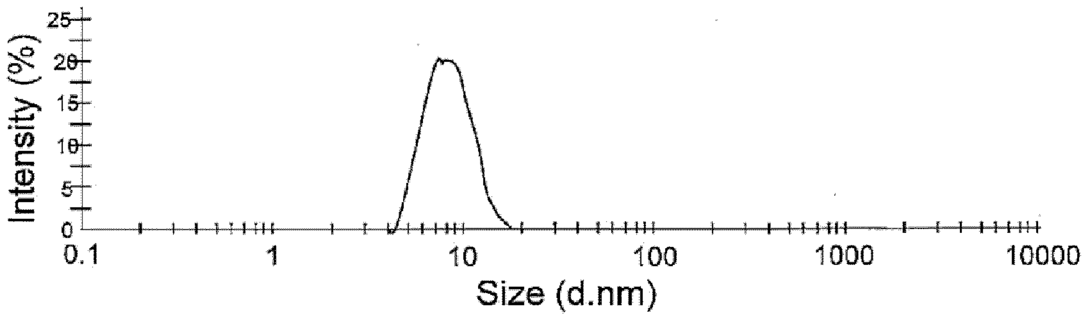


[FIG. 5]

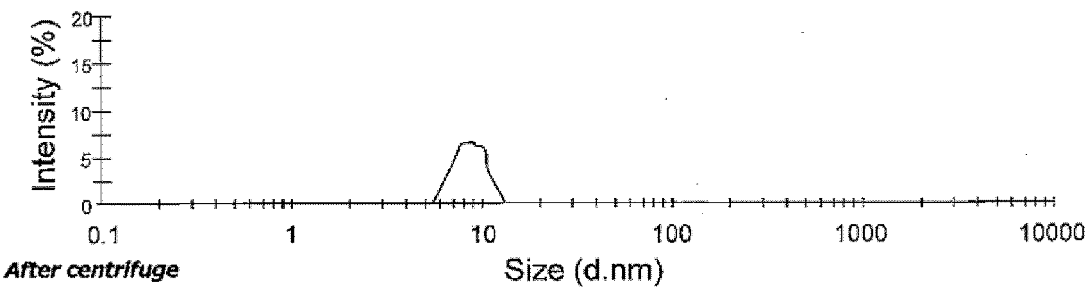


After centrifuge

[FIG. 6]

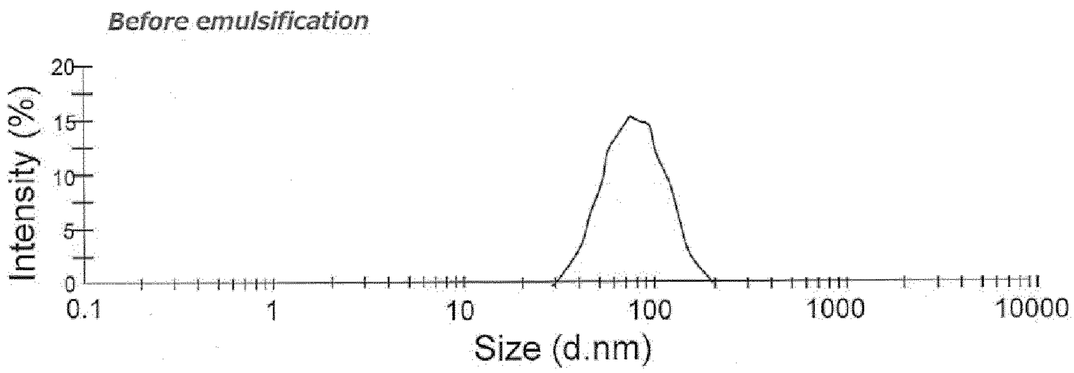
Before emulsification
Intensity (%)
20
15
10
5
0
0.1
1
10
100
1000
10000
Size (d.nm)

[FIG. 7]

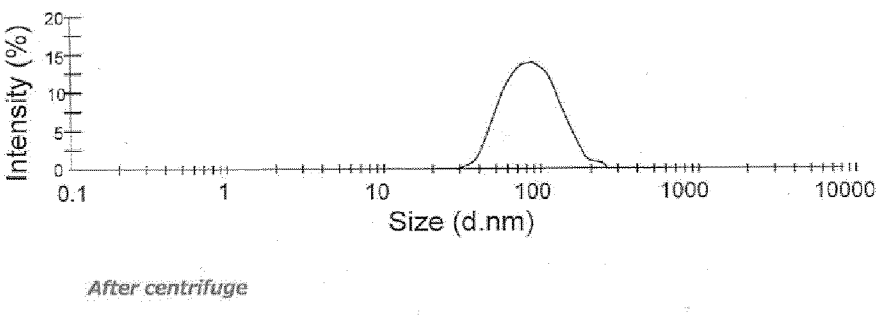
Intensity (%)
20
15
10
5
0
0.1
1
10
100
1000
10000
Size (d.nm)
After centrifuge

[FIG. 8]

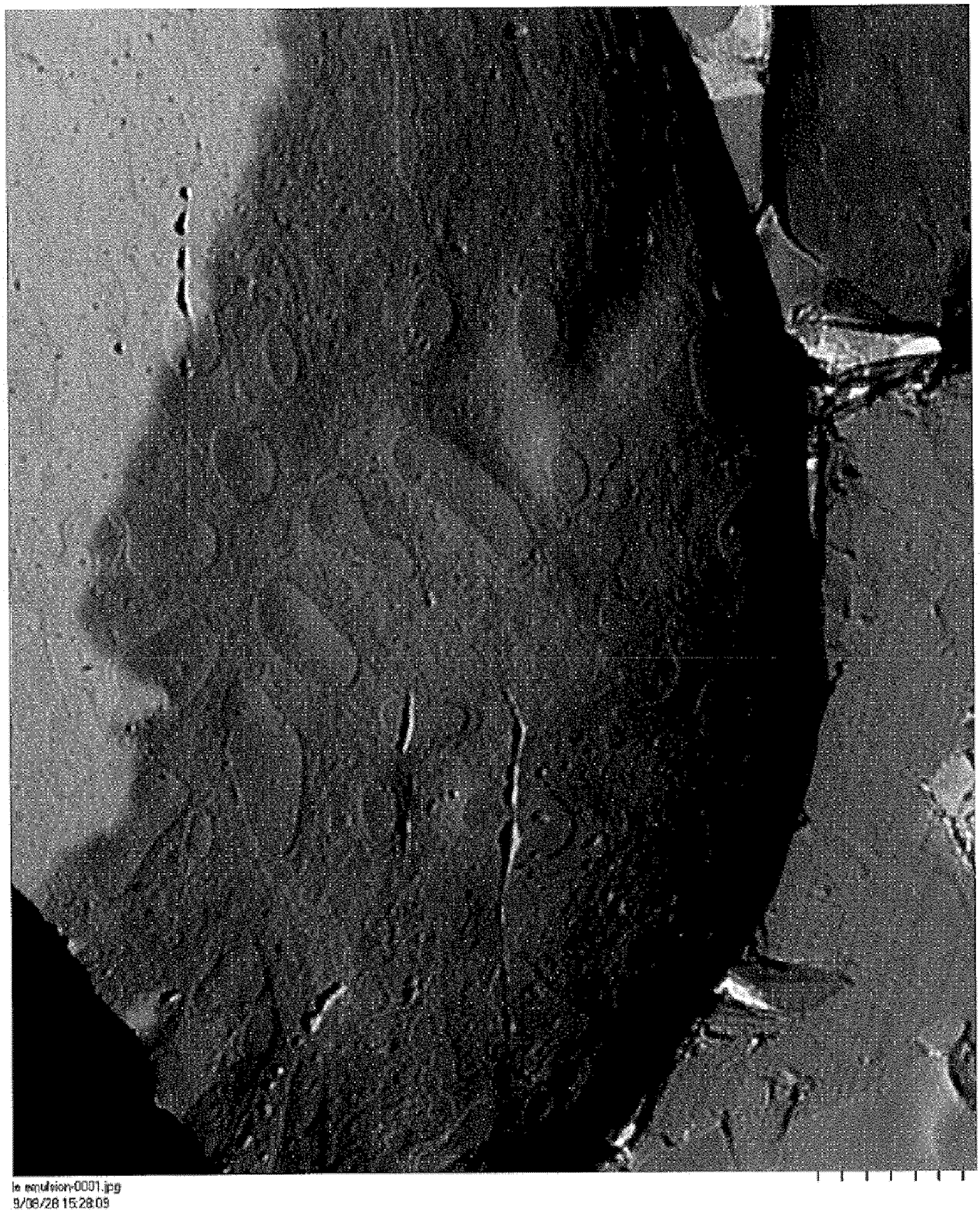
le emulsion-0001.jpg
9/08/28 15:28:09
9
=Hitachi TEM system.
nm/pixel at x 10.0k
1.0k
m-1
)'
0kV
\
=390 Tilt angle=-0.1

[FIG. 9]

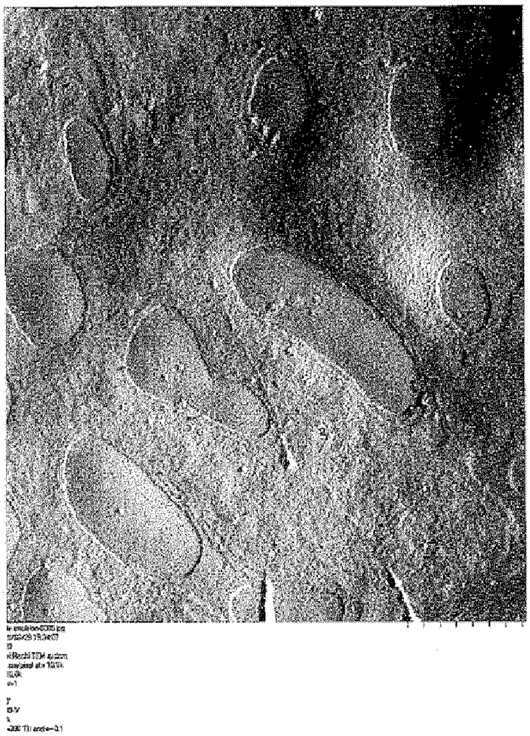

[FIG. 10]

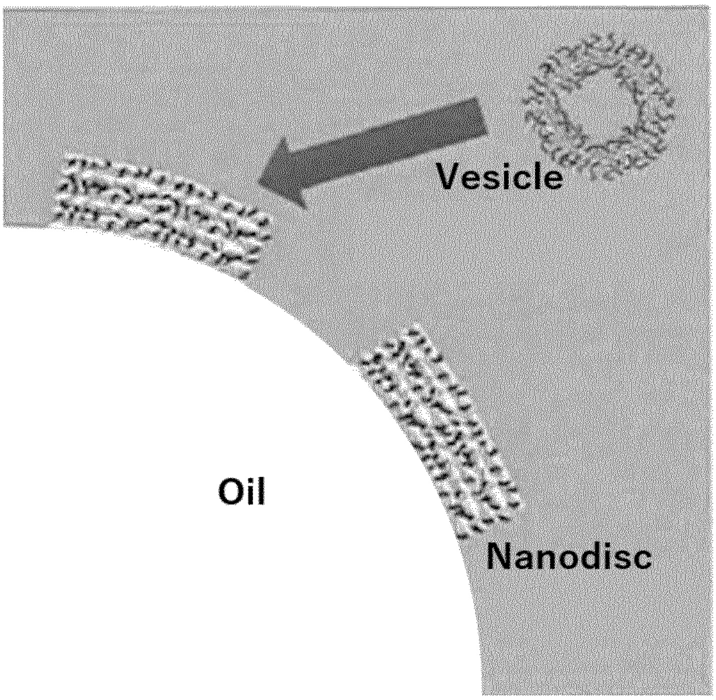

**INTERNATIONAL SEARCH REPORT**

International application No.
PCT/JP2021/008412

A. CLASSIFICATION OF SUBJECT MATTER

A61Q 19/00(2006.01)i; A61K 8/06(2006.01)i; A61K 8/34(2006.01)i; A61K 8/894(2006.01)i
FI: A61K8/06; A61K8/894; A61Q19/00; A61K8/34

According to International Patent Classification (IPC) or to both national classification and IPC

B. FIELDS SEARCHED

Minimum documentation searched (classification system followed by classification symbols)
A61Q19/00; A61K8/06; A61K8/34; A61K8/894

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>A | WO 2016/002751 A1 (SHISEIDO CO., LTD.) 07 January 2016 (2016-01-07) claims, paragraphs [0019], [0028], [0065], table 2, examples 5-8 | 1-6, 14-17, 19-20<br>7-13, 18 |
| X<br>A | WO 2011/129291 A2 (SHISEIDO CO., LTD.) 20 October 2011 (2011-10-20) claims, paragraph [0044], table 2, example 3 | 1-3, 14-16, 18-19<br>4-13, 17, 20 |
| X<br>A | JP 2016-060701 A (NOEVIR CO., LTD.) 25 April 2016 (2016-04-25) claims, paragraph [0037], table 1, example 1 | 1-2, 14-16, 19<br>3-13, 17-18, 20 |

☐ Further documents are listed in the continuation of Box C.　☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 April 2021 (23.04.2021) | 11 May 2021 (11.05.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2021/008412

| Patent Documents referred in the Report | Publication Date | Patent Family | Publication Date |
|---|---|---|---|
| WO 2016/002751 A1 | 07 Jan. 2016 | US 2017/0202758 A1 claims, paragraphs [0027], [0041], [0081], table 2, examples 5-8<br>EP 3162359 A1<br>CN 106456512 A<br>KR 10-2017-00188849 A | |
| WO 2011/129291 A2 | 20 Oct. 2011 | TW 2012-04400 A | |
| JP 2016-060701 A | 25 Apr. 2016 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2020036519 A
- WO 2010064678 A
- JP 2011195509 A
- JP 7323222 A
- JP 8239475 A
- JP 9175930 A
- JP 5121179 B
- JP 3137592 B

### Non-patent literature cited in the description

- **H. SAGITANI ; Y. HIRAI ; K. NABETA ; M. NAGAI.** Effect of Types of Polyols on Surfactant Phase Emulsification. *J. Jpn Oil Chem. Soc.,* 1986, vol. 35, 102-107
- **KEI WATANABE ; MIHARU NISHIDA ; KANAKO NISHIMURA ; YORIKO MUNE ; YUJI MATSUSHITA ; AYANO NAKAMURA ; KOJI TSUCHIYA ; HIDEKI SAKAI ; HEINZ HOFFMANN.** High Skin Hydration and Comfortable Texture of a Moisturizing Lotion Fulfilled by Controlling the Phase Sequence of a Vesicle/Micelle Complex. *J. Soc. Cosmet. Chem. Jpn.,* 2018, vol. 52 (4), 260-268